Europäisches Patentamt
European Patent Office
Office européen des brevets

(19) (11) Veröffentlichungsnummer: **0 053 325**
**A1**

(12) # EUROPÄISCHE PATENTANMELDUNG

(21) Anmeldenummer: **81109754.2**

(22) Anmeldetag: **19.11.81**

(51) Int. Cl.³: **A 61 K 31/41**
// C07D249/08

(30) Priorität: **28.11.80 DE 3044801**

(43) Veröffentlichungstag der Anmeldung: **09.06.82**
**Patentblatt 82/23**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Regel, Erik, Bergerheide 72a, D-5600 Wuppertal-1 (DE)**
Erfinder: **Büchel, Karl Heinz, Prof. Dr., Dabringhausener Strasse 42, D-5093 Burscheid (DE)**
Erfinder: **Plempel, Manfred, Dr., Pahlkestrasse 5, D-5600 Wuppertal-1 (DE)**

(54) **Triazol-Derivate als antimikrobielle Mittel.**

(57) Substituierten 1-Phenyl-2-triazolyl-1-penten-3-ole der Formel

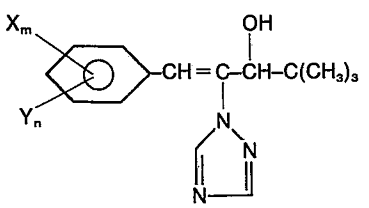

(I)

in welcher

X für Halogenalkyl, Halogenalkoxy, Halogenalkylthio, Alkylthio, Nitro, Hydroxy, Dialkylamino, Alkylcarbonyloxy oder gegebenenfalls substituiertes Benzyloxy steht,

Y für Halogen, Alkyl, Alkoxy, Cyano, gegebenenfalls substituiertes Phenyl oder Phenoxy steht,

m für ganze Zahlen von 1 bis 3 steht und

n für ganze Zahlen von 0 bis 4 steht, wobei m und n zusammen maximal für 5 stehen,

haben gute antimikrobielle, insbesondere antimykotische Eigenschaften.

EP 0 053 325 A1

BAYER AKTIENGESELLSCHAFT 5090 Leverkusen, Bayerwerk
Zentralbereich Si/W
Patente, Marken und Lizenzen II(Pha)

BEZEICHNUNG GEÄNDERT
siehe Titelseite

Antimikrobielle Mittel

Die vorliegende Erfindung betrifft die Verwendung von neuen substituierten 1-Phenyl-2-triazolyl-1-penten-3-olen als antimikrobielle Mittel, insbesondere als Antimykotika.

Es ist bereits bekannt geworden, daß bestimmte 4,4-Dimethyl-1-phenyl-2-triazolyl-1-penten-3-ole eine gute fungizide Wirksamkeit besitzen (vergleiche DE-OS 28 38 847). Die Wirkung dieser Verbindungen gegen humanpathogene Pilze ist jedoch nicht immer ganz befriedigend.

Es wurde gefunden, daß die neuen substituierten 1-Phenyl-2-triazolyl-1-penten-3-ole der allgemeinen Formel

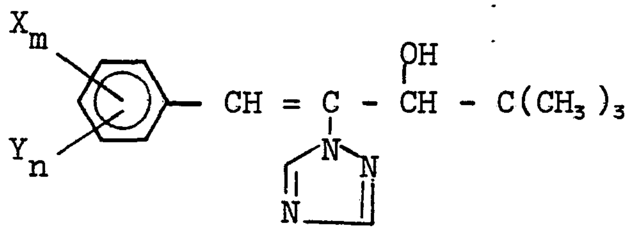

(I)

in welcher

X für Halogenalkyl, Halogenalkoxy, Halogenalkylthio, Alkylthio, Nitro, Hydroxy, Dialkylamino, Alkylcarbonyloxy oder gegebenenfalls substituiertes Benzyloxy steht,

Y für Halogen, Alkyl, Alkoxy, Cyano, gegebenenfalls substituiertes Phenyl oder Phenoxy steht,

m für ganze Zahlen von 1 bis 3 steht und

n für ganze Zahlen von 0 bis 4 steht, wobei m und n zusammen maximal für 5 stehen,

sowie deren physiologisch verträglichen Säureadditionssalze gute antimikrobielle, insbesondere antimykotische Eigenschaften aufweisen.

Die erfindungsgemäßen Verbindungen der Formel (I) kommen in den geometrischen Isomeren E (trans) und Z (cis) vor.

Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet

von zusammen) vor, stehen sie auf der entgegengesetzten Seite, liegt die Konfiguration E (abgeleitet von entgegen) vor.

Da außerdem ein asymmetrisches Kohlenstoffatom vorhanden ist, können die Verbindungen der Formel (I) in zwei optischen Isomerenformen vorliegen.

Die vorliegende Erfindung betrifft sowohl die einzelnen Isomeren als auch die Isomerengemische.

Ueberraschenderweise zeigen die erfindungsgemäß zu verwendenden substituierten 1-Phenyl-2-triazolyl-1-penten-3-ole eine bessere antimykotische Wirkung, insbesondere auch in-vivo gegen Candida, als die aus dem Stand der Technik bekannten 4,4-Dimethyl-1-phenyl-2-triazolyl-1-penten-3-ole, welche chemisch die naheliegendsten Verbindungen sind. Die erfindungsgemäße Verwendung der neuen Stoffe stellt somit eine Bereicherung der Pharmazie dar.

Die erfindungsgemäß zu verwendenden 1-Phenyl-2-triazolyl-1-penten-3-ole sind durch die Formel (I) allgemein definiert. In dieser Formel steht X vorzugsweise für Halogenalkyl, Halogenalkoxy und Halogenalkylthio mit jeweils 1 bis 2 Kohlenstoff- und bis zu 5 gleichen oder verschiedenen Halogenatomen, wie insbesondere Fluor- und Chloratomen; Alkylthio mit 1 bis 4 Kohlenstoffatomen; Nitro; Hydroxy; Dialkylamino mit 1 bis 4 Kohlenstoffatomen in jedem Alkylteil; Alkylcarbonyloxy mit 1 bis 4 Kohlenstoffatomen im Alkylteil; sowie für gegebenenfalls substituiertes Benzyloxy, wobei als Substituenten vorzugsweise genannt seien: Fluor, Chlor, Brom und Alkyl mit 1 bis 2 Kohlenstoffatomen.

Y steht vorzugsweise für Fluor, Chlor, Brom; Alkyl und Alkoxy mit jeweils 1 bis 4 Kohlenstoffatomen; Cyano; sowie für gegebenenfalls substituiertes Phenyl und Phenoxy, wobei als Substituenten vorzugsweise genannt seien: Fluor, Chlor, Brom und Alkyl mit 1 bis 2 Kohlenstoffatomen.

Der Index m steht vorzugsweise für die ganzen Zahlen 1 oder 2 und der Index n steht vorzugsweise für ganze Zahlen von 0 bis 3.

Besonders bevorzugt sind diejenigen Verbindungen der Formel (I), in denen X für Trifluormethyl, Difluorchlormethyl, Fluordichlormethyl, Trichlormethyl, 1,1,2-Trifluor-2-chlor-ethyl, Trifluormethoxy, Trifluormethylthio, 1,1,2-Trifluor-2-chlor-ethoxy und -ethylthio, Methylthio, Nitro, Hydroxy, Dimethylamino, Acetoxy, tert.-Butylcarbonyloxy, sowie für gegebenenfalls einfach oder zweifach, gleich oder verschieden durch Fluor, Chlor und Methyl substituiertes Benzyloxy steht; Y für Fluor, Chlor, Methyl, Ethyl, Isopropyl, tert.-Butyl, Methoxy, Isopropoxy, Cyano, sowie für gegebenenfalls einfach oder zweifach, gleich oder verschieden durch Fluor, Chlor und Methyl substituiertes Phenyl und Phenoxy steht; der Index m für die ganzen Zahlen 1 oder 2 steht; und der Index n für die ganzen Zahlen von 0 bis 2 steht.

Die erfindungsgemäß zu verwendenden Wirkstoffe sind noch nicht bekannt. Sie können jedoch gemäß einem eigenen Vorschlag hergestellt werden, indem man 1-Phenyl-2-triazolyl-1-penten-3-one der Formel

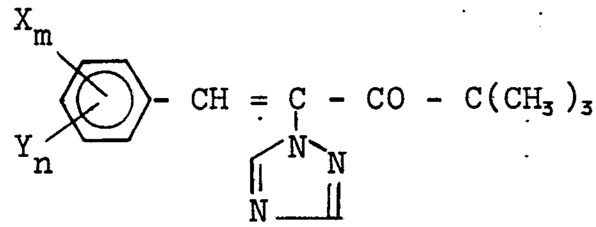 (II)

in welcher
X,Y,n und m die oben angegebene Bedeutung haben,

nach bekannten Methoden reduziert, wie z.B. durch Umsetzung mit komplexen Hydriden, wie insbesondere Natrium- und Calciumborhydrid, gegebenenfalls in Gegenwart eines polaren Lösungsmittels, wie beispielsweise Alkohole, bei Temperaturen zwischen 0 und 30°C; oder durch Umsetzung mit Aluminiumisopropylat in Gegenwart eines polaren organischen Lösungsmittels, wie beispielsweise Isopropanol, bei Temperaturen zwischen 20 und 120°C. Die Aufarbeitung erfolgt in üblicher Weise; gegebenenfalls wird das Salz hergesetellt. In manchen Fällen erweist es sich als vorteilhaft, die Verbindungen der Formel (I) über ihre Salze in reiner Form zu erhalten.

Bei der Durchführung der Reduktion können die Keto-Derivate der Formel (II) als E/Z-Isomerengemische oder als reine E- oder Z-Isomeren eingesetzt werden. Bei der Verwendung von Isomerengemischen als Ausgangsprodukte und von komplexen Hydriden als Reduktionsmittel, fallen die Endprodukte der Formel (I) vorzugsweise als E/Z-Isomerengemische an; bei der Verwendung von Aluminiumisopropylat als Reduktionsmittel wird lediglich das Z-Isomere reduziert. Eine Trennung in die reinen Isomeren ist allgemein auf übliche Art und Weise möglich , wie z. B. durch Kristallisation oder durch chromatographische Trennverfahren.

Ein eindeutiges Charakterisierungsmerkmal für die beiden geometrischen Isomeren ist die H[1]-Kernresonanz der zwei Triazol-Protonen. Die Differenz der beiden Shiftwerte für diese beiden Protonen ist in den E-Formen ungefähr doppelt so groß wie in den entsprechenden Z-Formen.

Die 1-Phenyl-2-triazolyl-1-penten-3-one der Formel (II) sind ebenfalls noch nicht bekannt. Sie können jedoch in bekannter Art und Weise erhalten werden, indem man Triazolyl-pinakolin der Formel

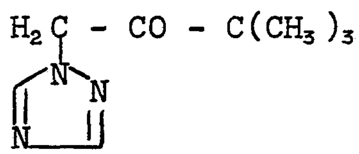 (III)

mit Aldehyden der Formel

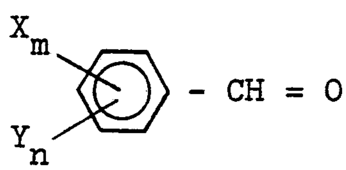 (IV)

in welcher

X,Y,m und n die oben angegebene Bedeutung haben,

in Gegenwart eines inerten organischen Lösungsmittels, wie beispielsweise Toluol, und in Gegenwart eines Katalysators, wie beispielsweise Piperidinacetat, bei Temperaturen zwischen 20 und 160°C, wie insbesondere bei der Siedetemperatur des jeweiligen Lösungsmittels, umsetzt. Dabei fallen die Verbindungen der Formel (II)

vorzugsweise als E/Z-Isomerengemische an. Eine Trennung in die reinen Isomeren ist auf übliche Art und Weise möglich, wie z.B. durch Kristallisation oder durch chromatographische Trennverfahren.

Zur Herstellung von physiologisch verträglichen Säureadditionssalzen der Verbindungen der Formel (I) kommen folgende Säuren infrage: Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Art und Weise, z.B. durch Abfiltrieren, isoliert und gegebenenfalls durch Waschen mit einem inerten organischen Lösungsmittel gereinigt werden.



BAD ORIGINAL

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bisphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabellen, Dragges, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen,

Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Oele, insbesondere Baumwollsaatöl, Erdnußöl, Meiskeimöl, Olivenöl, Ricinusöl und Sesaöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süßmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden , z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

(I-1) 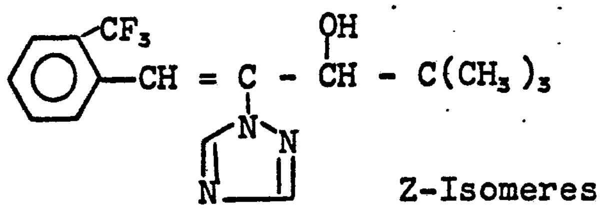

Z-Isomeres

(II-1) 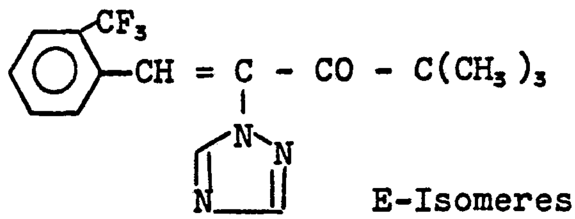

E-Isomeres

44,1g (0,1365 Mol) 4,4-Dimethyl-2-(1,2,4-triazol-1-yl)-1-(2-trifluormethylphenyl)-1-penten-3-on als E/Z-Isomerengemisch und 27,9g (0,1365 Mol) Aluminiumisopropylat werden 7 Stunden in 350 ml siedendem Isopropanol erhitzt; hierbei wird über eine 30 cm-Vigreux-kolonne kontinuierlich Isopropanol und Aceton abdestilliert bis im Destillat kein Aceton mehr nachzuweisen ist. Danach wird die Lösung mit Eis/Salzsäure zersetzt. Nach Extraktion mit Methylenchlorid wird die organische Phase über Natriumsulfat getrocknet, filtriert und im Vakuum eingedampft. Die verbleibende halbkristalline Masse wird über Kieselgel-60 (Merck)/Chloroform chromatographiert. Die ersten Fraktionen ergeben nach Abdampfen des Lösungsmittels 10,8g (24% d.Theorie) E-isomeres 4,4-Dimethyl-2-(1,2,4-triazol-1-yl)1-(2-trifluormethylphenyl)-1-penten-3-on vom Schmelzpunkt 82°C.

Die nächsten Fraktionen ergeben nach Abdampfen des Lösungsmittels 12,8g (28 % der Theorie) Z-isomeres 4,4-Dimethyl-2-(1,2,4-triazol-1-yl)-1-(2-trifluormethylphenyl)-1-penten-3-ol vom Schmelzpunkt 136°C.

Herstellung des Ausgangsproduktes

(II-2) 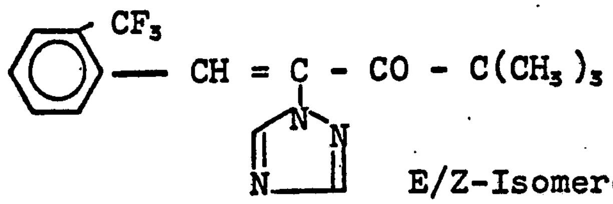

E/Z-Isomerengemisch

25,2g (0,15 Mol) 3,3-Dimethyl-1-(1,2,4-triazol-1-yl)-butan-2-on und 26,1g (0,15 Mol) 2-Trifluorbenzaldehyd werden in 350 ml Toluol mit 9 g Essigsäure und 3,6 ml Dimethylmorpholin 20 Stunden unter Rückfluß erhitzt und das Reaktionswasser azeotrop entfernt. Die Toluollösung wird mit Wasser gewaschen, über Natriumsulfat getrocknet, filtriert und im Vakuum eingedampft. Man erhält 44,9g (93 % der Theorie) 4,4-Dimethyl-2-(1,2,4-triazol-1-yl)-1-(2-trifluormethylphenyl)-1-penten-3-on als E/Z-Isomerengemisch vom Brechungsindex $n_D^{20}$=1,5113.

Beispiel 2

(I-2) 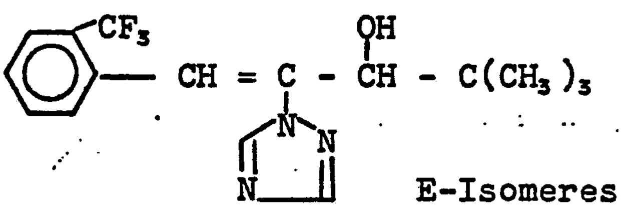

E-Isomeres

10,7g (0,0345 Mol) E-isomeres 4,4-Dimethyl-2-(1,2,4-triazol-1-yl)-1-(2-trifluormethylphenyl)-1-penten-3-on (vgl.Beispiel 1) und 2,45 g(0,0233 Mol) Calciumchlorid werden in 150 ml Isopropanol bei -5°C mit einer Lösung von 0,85 g (0,0242 Mol) Natriumboranat in 20 ml Wasser tropfenweise versetzt.

Nach 18 Stunden werden 20 ml Aceton zugetropft. Nach Abdampfen des Lösungsmittels wird der verbleibende Rückstand in Wasser eingerührt und mit Methylenchlorid extrahiert, die organische Phase wird abgetrennt, über Natriumsulfat getrocknet, filtriert und im Vakuum eingedampft. Die Kristallmasse wird mit Diisopropylether verrührt und abgesaugt. Man erhält 7,2g (66,7% der Théorie) E-isomeres 4.4-Dimethyl-2-(1,2,4-triazol-1-yl)-1-(2-trifluormethylphenyl)-1-penten-3-ol vom Schmelzpunkt 165°C.

<u>Beispiel 3</u>

(I-3) 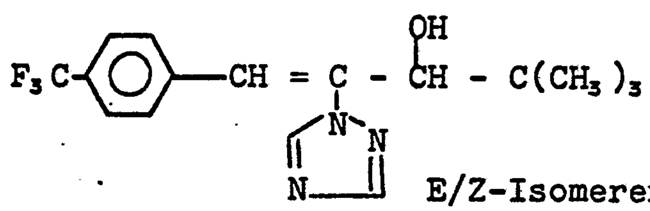

E/Z-Isomerengemisch

61,5g (0,19 Mol) 4,4-Dimethyl-2-(1,2,4-triazol-1-yl)-1-(4-trifluormethylphenyl)-1-penten-3-on als E/Z-Isomerengemisch und 14,1g (0,1275 Mol) Calciumchlorid werden in 400 ml Isopropanol bei -5°C mit einer Lösung von 5,05 g (0,13 Mol) Natriumboranat in 100 ml Wasser tropfenweise versetzt. Nach 15 Stunden werden 60 ml

Aceton zugetropft. Nach Abdampfen des Lösungsmittels wird der verbleibende Rückstand in Wasser eingerührt und mit Essigsäureethylester extrahiert. Die Lösung wird über Natriumsulfat getrocknet, filtriert und im Vakuum eingedampft. Man erhält 60,3g (97 % der Theorie) 4,4-Dimethyl-2-(1,2,4-triazol-1-yl)-1-(4-trifluormethylphenyl)-1-penten-3-ol als E/Z-Isomerengemisch vom Schmelzpunkt 54-55°C.

In entsprechender Weise werden die folgenden Verbindungen der allgemeinen Formel (I) erhalten:

<u>T a b e l l e 1</u>

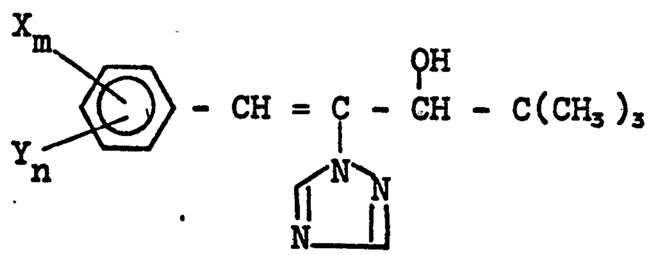

(I)

| Bsp. Nr. | $X_m$ | $Y_n$ | Schmelzpunkt(°C) |
|---|---|---|---|
| I-4 | 4-OH | - | 168(E/Z-Gemisch) |
| I-5 | 4-OH | 3-$OCH_3$ | 136(E/Z-Gemisch) |
| I-6 | 2-OH | 3,5-$Cl_2$ | 158(E/Z-Gemisch) |
| I-7 | 4-$N(CH_3)_2$ | - | 140(E/Z-Gemisch) |
| I-8 | 4-O-$CH_2$- 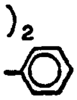 | - | 109(E/Z-Gemisch) |
| I-9 | 4-$NO_2$ | - | 126(Z-Isomeres) |
| I-10 | 4-$NO_2$ | - | 188(E-Isomeres) |
| I-11 | 4-$CF_3$ | 2-Cl | 142(Z-Isomeres) |
| I-12 | 4-$CF_3$ | 2-Cl | 134(E-Isomeres) |
| I-13 | 4-$OCF_3$ | - | 113(Z-Isomeres) |
| I-14 | 4-$OCF_3$ | - | 108(E-Isomeres) |
| I-15 | 4-$SCF_3$ | - | 95(Z-Isomeres) |
| I-16 | 4-$SCF_3$ | - | Oel(E-Isomeres) |
| I-17 | 4-$CF_3$ | - | 128(Z-Isomeres) |
| I-18 | 4-$CF_3$ | - | 130(E-Isomeres) |
| I-19 | 4-$CF_3$ | 3-Cl | 130(Z-Isomeres) |
| I-20 | 4-$CF_3$ | 3-Cl | 141(E-Isomeres) |
| I-21 | 4-$OCF_3$ | 3-Cl | 110(Z-Isomeres) |
| I-22 | 4-$OCF_3$ | 3-Cl | 91(E-Isomeres) |
| I-23 | 3-$NO_2$ | 4-Cl | 150(Z-Isomeres) |
| I-24 | 3-$NO_2$ | 4-Cl | Harz(E-Isomeres) |
| I-25 | 3-$CF_3$ | - | 120(Z-Isomeres) |
| I-26 | 3-$CF_3$ | - | $n_D^{20}$ = 1,5080 (E-Isomeres) |

Entsprechend Beispiel 1 und entsprechend dem im Text beschriebenen Verfahren werden die folgenden Ausgangsstoffe der Formel (II) erhalten:

Tabelle 2

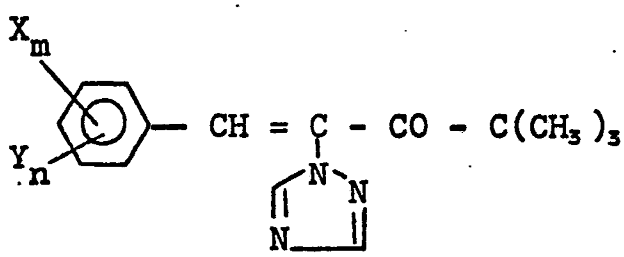

(II)

| Bsp.Nr. | $X_m$ | $Y_n$ | Schmelzpunkt(°C)bzw. Brechungsindex($n_D^{20}$) |
|---|---|---|---|
| II-2 | 4-OH | - | 186(E-Isomeres) |
| II-3 | 4-$NO_2$ | - | 125(E-Isomeres) |
| II-4 | 4-OH | 3-$OCH_3$ | 134(E/Z-Gemisch) |
| II-5 | 2-OH | 3,5-$Cl_2$ | 210(E/Z-Gemisch) |
| II-6 | 4-$N(CH_3)_2$ | - | 112(E/Z-Gemisch) |
| II-7 | 4-O-$CH_2$- 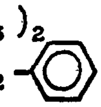 | - | 120(E/Z-Gemisch) |
| II-8 | 4-$CF_3$ | - | 119(E-Isomeres) |
| II-9 | 4-$CF_3$ | 2-Cl | 1,5134(E/Z-Gemisch) |
| II-10 | 4-O-$CF_3$ | - | 98(E-Isomeres) |
| II-11 | 4-$OCF_3$ | - | 1,5145(E/Z-Gemisch) |
| II-12 | 4-$CF_3$ | 3-Cl | 1,5236(E/Z-Gemisch) |
| II-13 | 4-$OCF_3$ | 3-Cl | 1,5195(E/Z-Gemisch) |
| II-14 | 3-$CF_3$ | - | 1,5220(E/Z-Gemisch) |
| II-15 | 3-$CF_3$ | - | 1,5168(E-Isomeres) |

Verwendungsbeispiele

In den nachfolgenden Beispielen werden die nachstehend angegebenen Verbindungen als Vergleichssubstanzen eingesetzt:

(A) 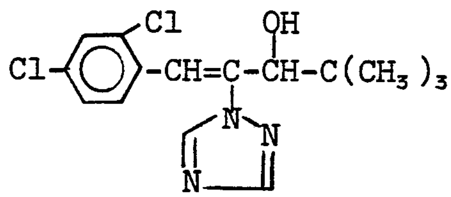

(B) 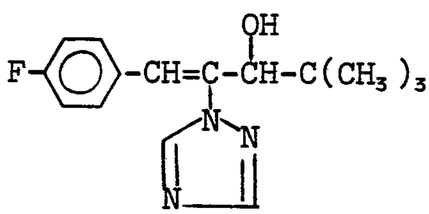

Beispiel A

Antimykotische in-vitro-Wirksamkeit

Versuchsbeschreibung:

Die in-vitro-Prüfungen wurden im Reihenverdünnungstest mit Keiminokula von durchschnittlich 5 x $10^4$ Keimen/ml Substrat durchgeführt. Als Nährmedium dienten

a) für Dermatophyten und Schimmelpilze: Sabourand's milieu d'épreuve

b) für Hefen: Fleischextrakt-Traubenzucker-Bouillon.

Die Bebrütungstemperatur betrug 20°C, die Bebrütungsdauer lag bei 24 bis 96 Stunden.

In diesem Test zeigen insbesondere die Beispiele 2,3, 10, 12,14,16,18,20 und 22 eine bessere antimykotische Wirkung als die aus dem Stand der Technik bekannten Verbindungen (A) und (B).

## Beispiel B

### Antimykotische in-vivo-Wirksamkeit (oral) bei Mäuse-Candidose

Versuchsbeschreibung:

Mäuse vom Typ SPF-$CF_1$ wurden intravenös mit 1 - 2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 50 - 100mg/kg Körpergewicht der Präparate oral behandelt.

Ergebnis:

Unbehandelte Tiere starben 3 bis 6 Tage post infektionem. Die Ueberlebensrate am 6.Tag post infektionem betrug bei unbehandelten Kontrolltieren etwa 5 %.
In diesem Test zeigen z.B. die erfindungsgemäßen Verbindungen 3, 9. 10, 12, 14 und 18 eine bessere Wirkung als die aus dem Stand der Technik bekannten Verbindungen (A) und (B).

Patentansprüche

1) Antimykotisches Mittel, gekennzeichnet durch einen Gehalt an mindestens einem substituierten 1-Phenyl-2-triazolyl-1-penten-3-ol der allgemeinen Formel

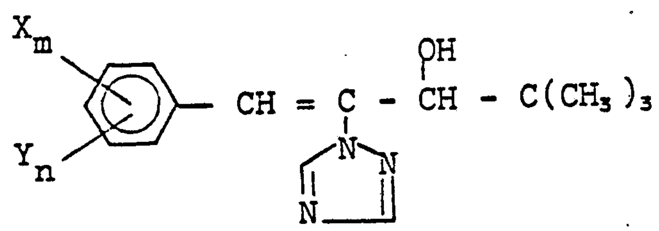

(I)

in welcher

X für Halogenalkyl, Halogenalkoxy, Halogenalkylthio, Alkylthio, Nitro, Hydroxy, Dialkylamino, Alkylcarbonyloxy oder gegebenenfalls substituiertes Benzyloxy steht,

Y für Halogen, Alkyl, Alkoxy, Cyano, gegebenenfalls substituiertes Phenyl oder Phenoxy steht,

m für ganze Zahlen von 1 bis 3 steht und

n für ganze Zahlen von 0 bis 4 steht, wobei m und n zusammen maximal für 5 stehen,

und/oder deren physiologisch verträglichen Säureadditionssalzen.

2) Antimykotisches Mittel, gekennzeichnet durch einen Gehalt an mindestens einem substituierten 1-Phenyl-2-triazolyl-1-penten-3-ol der allgemeinen Formel I,

in welcher X für Halogenalkyl, Halogenalkoxy und Halogenalkylthio mit jeweils 1 oder 2 Kohlenstoffatomen und 1 bis 5 gleichen oder verschiedenen Halogenatomen, für Alkylthio mit 1 bis 4 Kohlenstoffatomen, Nitro, Hydroxy, Dialkylamino mit 1 bis 4 Kohlenstoffatomen in jedem Alkylteil, Alkylcarbonyloxy mit 1 bis 4 Kohlenstoffatomen im Alkylteil und für gegebenenfalls durch Fluor, Chlor, Brom und/oder Alkyl mit 1 bis 2 Kohlenstoffatomen substituiertes Benzyl steht, Y für Fluor, Chlor, Brom, Alkyl mit 1 bis 4 Kohlenstoffatomen, Alkoxy mit 1 bis 4 Kohlenstoffatomen, Cyano, gegebenenfalls durch Fluor, Chlor, Brom und/oder Alkyl mit 1 oder 2 Kohlenstoffatomen substituiertes Phenyl oder für gegebenenfalls durch Fluor, Chlor, Brom und/oder Alkyl mit 1 oder 2 Kohlenstoffatomen substituiertes Phenoxy steht, m für die ganzen Zahlen 1 oder 2 steht und n für ganze Zahlen von O bis 3 steht.

3) Antimykotisches Mittel, gekennzeichnet durch einen Gehalt an mindestens einem substituierten 1-Phenyl-2-triazolyl-1-penten-3-ol der allgemeinen Formel I, in welcher X für Trifluirmethyl, Difluorchlormethyl, Fluordichlormethyl, Trichlormethyl, 1,1,2-Trifluor-2-chlor-ethyl, Trifluormethoxy, Trifluormethylthio, 1,1,2-Trifluor-2-chlor-ethoxy und -ethylthio, Methylthio, Nitro, Hydroxy, Dimethylamino, Acetoxy, tert.-Butylcarbonyloxy, sowie für gegebenenfalls einfach oder zweifach, gleich oder verschieden durch Fluor, Chlor und Methyl substituiertes Benzyloxy, Y für Fluor, Chlor, Methyl, Ethyl, Isopropyl, tert.-Butyl,

Methoxy, Isopropoxy, Cyano, sowie für gegebenenfalls einfach oder zweifach, gleich oder verschieden durch Fluor, Chlor und Methyl substituiertes Phenyl und Phenoxy, der Index m für die ganzen Zahlen 1 oder 2 und der Index n für die ganzen Zahlen von 0 bis 2 steht.

4) Verfahren zu Herstellung eines antimykotischen Mittels, dadurch gekennzeichnet, daß man substituierte 1-Phenyl-2-triazolyl-1-penten-3-ole und/oder deren physiologisch verträglichen Säureadditionssalze gemäß der Formel in Anspruch 1 mit inerten, nichttoxischen, pharmazeutisch geeigneten Trägerstoffen vermischt.

5) Verfahren zur Behandlung von Mykosen, dadurch gekennzeichnet, daß man substituierte 1-Phenyl-2-triazolyl-1-penten-3-ole und/oder deren physiologisch verträglichen Säureadditionssalze gemäß der Formel in Anspruch 1 Menschen oder Tieren appliziert, die an Mykosen erkrankt sind.

0053325

Europäisches Patentamt

# EUROPÄISCHER RECHERCHENBERICHT

Nummer der Anmeldung

EP 81 10 9754

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A - 2 920 437 (BAYER A.G.)<br>* Insgesamt * | 1-5 |
| X | GB - A - 2 046 260 (SUMITOMO CHEMICAL CO.)<br>* Insgesamt * | 1-5 |
| Y | EP - A - 0 015 387 (BAYER A.G.)<br>* Ansprüche * | 1-5 |
| Y | FR - A - 2 327 242 (I.C.I.)<br>* Ansprüche * | 1-5 |
| P | EP - A - 0 022 975 (BAYER A.G.)<br>* Seiten 1-6 * | 1-5 |
| P | EP - A - 0 028 363 (BASF A.G.)<br>* Seiten 1-4 * | 1-5 |
| DX | GB - A - 2 004 276 (SUMITOMO CHEMICAL CO.)<br>* Insgesamt * | 1-5 |
| A | US - A - 4 182 862 (H.F. CHAN)<br>* Spalten 1-4 * | 1 |

KLASSIFIKATION DER ANMELDUNG (Int. Cl.)

A 61 K 31/41
C 07 D 249/08

RECHERCHIERTE SACHGEBIETE (Int. Cl.)

C 07 D 249/00
A 01 N 43/00
A 61 K 31/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 15-02-1982 | BRIGHENTI |